# EUROPEAN PATENT APPLICATION

(11) **EP 4 748 923 A1**
(43) Date of publication of application: **27.05.2026**
(21) Application number: 24842199.2
(22) Date of filing: 03.07.2024
(51) Int. Cl.: C12N 1/21, C12N 15/70, C12N 15/60, C12P 13/10

(54) **METHOD FOR PRODUCING L-ARGININE USING RECOMBINANT ESCHERICHIA COLI, AND RECOMBINANT BACTERIUM USED THEREIN**

(30) Priority: 18.07.2023 CN 202310879978; 18.07.2023 CN 202310879981
(71) Applicant: Ningxia Eppen Biotech Co. Ltd, Ningxia 750100 (CN)
(72) Inventor: XIE, Xixian, Yinchuan, Ningxia 750100 (CN); JIANG, Shuai, Yinchuan, Ningxia 750100 (CN); WU, Heyun, Yinchuan, Ningxia 750100 (CN); MA, Qian, Yinchuan, Ningxia 750100 (CN); LI, Ran, Yinchuan, Ningxia 750100 (CN); MENG, Gang, Yinchuan, Ningxia 750100 (CN); ZHAO, Chunguang, Yinchuan, Ningxia 750100 (CN); WEI, Aiying, Yinchuan, Ningxia 750100 (CN); JIA, Huiping, Yinchuan, Ningxia 750100 (CN)
(74) Representative: Winter, Brandl - Partnerschaft mbB
(86) International application number: PCT/CN2024/103336
(87) International publication number: WO 2025/016203

(57) **Abstract**

The disclosure discloses a method for producing L-arginine using recombinant enterobacteria and the recombinant strains used therein. This disclosure falls within the field of genetic engineering technology, specifically relating to a method for producing L-arginine using recombinant enterobacteria and the recombinant strains employed. The recombinant enterobacteria of the disclosure are obtained by overexpressing a cyanate transporter-encoding gene in recipient enterobacteria, or by overexpressing a carbonic anhydrase-encoding gene in recipient enterobacteria. The cyanate transporter is a protein with the amino acid sequence of SEQ ID No.4, and the carbonic anhydrase is a protein with the amino acid sequence of SEQ ID No.5. By regulating the expression of the cyanate transporter or carbonic anhydrase, the arginine yield is significantly increased, providing a new approach for synthesizing bio-based L-arginine and holding broad application potential and market prospects.

## Description

### CROSS-REFERENCE TO RELATED APPLICATION

This application claims the priority of Chinese patent applications filed with the China National Intellectual Property Administration on July 18, 2023, with application numbers 202310879981.0 and an disclosure title of "Method for Producing L-Arginine Using Escherichia coli with Overexpressed cynX Gene and Recombinant Bacterium Used Thereof," and application number 202310879978.9 with an disclosure title of "Method for Producing L-Arginine Using Escherichia coli with Overexpressed cynT Gene and Recombinant Bacterium Used Thereof." The entire contents of these applications are incorporated herein by reference.

### TECHNICAL FIELD

This disclosure falls within the field of genetic engineering technology, specifically relating to a method for producing L-arginine using recombinant Escherichia coli and the recombinant strain used therein.

### BACKGROUND

L-arginine has a wide range of applications in fields such as medicine, industry, food, cosmetics, and animal husbandry, holding significant economic and social value.

Currently, the production methods of L-arginine mainly include microbial fermentation and protein hydrolysis. Compared to protein hydrolysis, microbial fermentation offers advantages such as a wide range of raw material sources, relatively simple production processes, relatively minor environmental impacts, and high product purity, making it more suitable for large-scale industrial production.

The selection and breeding of efficient strains with the ability to produce L-arginine are crucial for the industrial application of microbial fermentation. Currently, there are two primary methods for selecting and breeding L-amino acid-producing strains: 1) irrational mutagenesis screening: this method mainly involves mutagenizing wild-type chassis microorganisms through physical or chemical means, combined with screening methods based on resistance to structural analogs of L-arginine, to select mutagenized strains that exhibit antagonistic effects against L-arginine. After multiple rounds of mutagenesis, excellent production strains with the ability to synthesize L-arginine are ultimately screened out; 2) rational metabolic engineering modification: this method primarily utilizes efficient gene editing technologies to systematically modify the L-arginine synthesis network in chassis microorganisms through metabolic engineering, aiming to maximize the redirection of carbon metabolic flux towards the L-arginine synthesis pathway. This includes blocking L-arginine degradation pathways, relieving feedback inhibition regulatory mechanisms of key enzymes in the synthesis pathway, enhancing the supply of the precursor carbamoyl phosphate, optimizing the balance of coenzyme supply in chassis cells, and modifying the L-arginine transmembrane transport system. With the rapid development of genetic engineering technologies, the method of constructing efficient L-arginine-producing strains through rational metabolic engineering modification has gradually replaced mutagenesis screening and breeding methods, becoming the mainstream approach for selecting and breeding efficient and stable L-arginine-producing strains.

Improving the L-arginine production performance of chassis microorganisms is an ongoing goal of rational metabolic engineering breeding. During the systematic reconstruction of the metabolic network of chassis microorganisms, enhancing or weakening the expression intensity of target genes is a common strategy to improve L-arginine production performance. For example, weakening the expression of one or several genes involved in L-arginine degradation, one or several genes in competitive pathways for L-arginine synthesis, or genes participating in carbon-nitrogen flux redistribution significantly promotes L-arginine synthesis. Additionally, increasing the expression of key enzymes in the L-arginine synthesis pathway, enhancing the expression of membrane proteins responsible for exporting the target product, and increasing the expression of key enzymes in the precursor synthesis pathway of the target product also have significant beneficial effects on improving L-arginine synthesis in chassis microorganisms.

The cynX gene encodes a cyanate transporter, and the impact of enhancing cynX gene expression intensity on L-arginine production has not been studied. In particular, there have been no reports on the production of L-arginine using Enterobacteriaceae bacteria overexpressing the cynX gene.

The cynT gene encodes carbonic anhydrase, which catalyzes the reaction between CO₂ and H₂O to synthesize H₂CO₃. The impact of enhancing cynT gene expression intensity on L-arginine production has not been studied, and in particular, there have been no reports on the production of L-arginine using Escherichia coli overexpressing the cynT gene.

### SUMMARY

The problem to be solved by this disclosure is how to enhance the ability of microorganisms to produce L-arginine.

In order to address the aforementioned problem, this disclosure provides a recombinant enterobacterium.

The recombinant enterobacterium provided by this disclosure is either recombinant enterobacterium A or recombinant enterobacterium B. Recombinant enterobacterium A is a recombinant strain obtained by overexpressing the cyanate transporter-encoding gene in the recipient enterobacterium; recombinant enterobacterium B is a recombinant strain obtained by overexpressing the carbonic anhydrase-encoding gene in the recipient enterobacterium.

In the aforementioned recombinant enterobacteria, the cyanate transporter gene is derived from Escherichia coli, and the carbonic anhydrase gene is also derived from Escherichia coli.

In the aforementioned recombinant enterobacteria, the cyanate transporter is any of the following proteins:
A1) a protein encoded by the cyanate transporter-encoding gene with the amino acid sequence set forth in SEQ ID No.4;
A2) a protein with more than 80% identity to the protein in A1) and possessing cyanate transporter activity, obtained by substituting, deleting, and/or adding amino acid residues in the amino acid sequence of A1);
A3) a fusion protein with cyanate transporter activity obtained by attaching a tag to the N-terminus and/or C-terminus of A1) or A2).

In the aforementioned recombinant enterobacteria, the carbonic anhydrase is any of the following proteins:
B1) a protein encoded by the carbonic anhydrase-encoding gene with the amino acid sequence set forth in SEQ ID No.5;
B2) a protein with more than 75% identity to the protein in B1) and possessing carbonic anhydrase activity, obtained by substituting, deleting, and/or adding amino acid residues in the amino acid sequence of B1);
B3) a fusion protein with carbonic anhydrase activity obtained by attaching a tag to the N-terminus and/or C-terminus of B1) or B2).

The protein with the amino acid sequence set forth in SEQ ID No.4 mentioned above is a cyanate transporter (abbreviated as cynX).

In a specific embodiment, the amino acid sequence of the cyanate transporter is shown as follows:

MLLVLVLIGLNMRPLLTSVGPLLPQLRQASGMSFSVAALLTALPVVTMGGLALAGSWL
HQHVSERRSVAISLLLIAVGALMRELYPQSALLLSSALLGGVGIGIIQAVMPSVIKRRFQQRTP
LVMGLWSAALMGGGGLGAAITPWLVQHSETWYQTLAWWALPAVVALFAWWWQSAREVA
SSHKTTTTPVRVVFTPRAWTLGVYFGLINGGYASLIAWLPAFYIEIGASAQYSGSLLALMTL
GQAAGALLMPAMARHQDRRKLLMLALVLQLVGFCGFIWLPMQLPVLWAMVCGLGLGGA
FPLCLLLALDHSVQPAIAGKLVAFMQGIGFIIAGLAPWFSGVLRSISGNYLMDWAFHALCVV
GLMIITLRFAPVRFPQLWVKEA (SEQ ID No.4).

The protein with the amino acid sequence set forth in SEQ ID No.5 mentioned above is carbonic anhydrase (abbreviated as cynT).

In a specific embodiment, the amino acid sequence of the carbonic anhydrase is as follows:

MKEIIDGFLKFQREAFPKREALFKQLATQQSPRTLFISCSDSRLVPELVTQREPGDLFVIR
NAGNIVPSYGPEPGGVSASVEYAVAALRVSDIVICGHSNCGAMTAIASCQCMDHMPAVSHW
LRYADSARVVNEARPHSDLPSKAAAMVRENVIAQLANLQTHPSVRLALEEGRIALHGWVY
DIESGSIAAFDGATRQFVPLAANPRVCAIPLRQPTAA (SEQ ID No.5).

In the aforementioned recombinant enterobacteria, the encoding gene of the cyanate transporter can be any of the following:
C1) a DNA molecule with the nucleotide sequence set forth in SEQ ID No.2;
C2) a DNA molecule encoding the cyanate transporter with more than 80% identity to the nucleotide sequence defined in C1);
C3) a DNA molecule that hybridizes under stringent conditions with the nucleotide sequence defined in either C1) or C2) and encodes the cyanate transporter.

In the aforementioned recombinant enterobacteria, the encoding gene of the carbonic anhydrase can be any of the following:
D1) a DNA molecule with the nucleotide sequence set forth in SEQ ID No.3;
D2) a DNA molecule encoding the carbonic anhydrase with more than 75% identity to the nucleotide sequence defined in D1);
D3) a DNA molecule that hybridizes under stringent conditions with the nucleotide sequence defined in either D1) or D2) and encodes the carbonic anhydrase.

Those skilled in the art can readily mutate the nucleotide sequences encoding the cyanate transporter or carbonic anhydrase of the present disclosure using known methods, such as directed evolution and point mutation. Nucleotides that have been artificially modified and exhibit 75% or higher identity to the nucleotide sequences of the cyanate transporter or carbonic anhydrase of the present disclosure are considered derived from and equivalent to the sequences of the present disclosure, provided they encode a cyanate transporter or carbonic anhydrase and possess the functions of a cyanate transporter or carbonic anhydrase.

The term "identity" as used herein refers to sequence similarity with a native nucleic acid sequence. "Identity" includes nucleotide sequences that have 75% or higher, or 85% or higher, or 90% or higher, or 95% or higher identity to the nucleotide sequences encoding the cyanate transporter or carbonic anhydrase of the present disclosure. Identity can be evaluated visually or using computer software. Using computer software, the identity between two or more sequences can be expressed as a percentage (%), which can be used to assess the identity between related sequences.

The aforementioned identity of 75% or more can be 80%, 85%, 90%, or 95% or more.

In the aforementioned recombinant Enterobacteriaceae, the expression level of the cyanate transporter-encoding gene or carbonic anhydrase-encoding gene in the recombinant Enterobacteriaceae is higher than that in the recipient Enterobacteriaceae.

In the aforementioned recombinant Enterobacteriaceae, the overexpression of the cyanate transporter or carbonic anhydrase-encoding gene in the recipient Enterobacteriaceae can be achieved by replacing the ygaY gene of the recipient Enterobacteriaceae with the cyanate transporter or carbonic anhydrase-encoding gene.

The method for replacing the ygaY gene of the recipient Enterobacteriaceae with the cyanate transporter or carbonic anhydrase-encoding gene includes integrating the cyanate transporter or carbonic anhydrase-encoding gene into the ygaY gene locus of the recipient Enterobacteriaceae and knocking out the ygaY gene of the recipient Enterobacteriaceae.

In the aforementioned recombinant Enterobacteriaceae, the predicted transporter ygaY gene encodes any one of the following proteins:
H1) a protein encoded by the predicted transporter-encoding gene ygaY with an amino acid sequence of SEQ ID No.6;
H2) a protein obtained by substituting and/or deleting and/or adding amino acid residues from the amino acid sequence of H1), which has more than 80% identity to the protein of H1) and possesses predicted transporter protein activity;
H3) a fusion protein obtained by linking a tag to the N-terminus and/or C-terminus of H1) or H2), which possesses predicted transporter protein activity.

In a specific embodiment, the encoded amino acid sequence of ygaY is:

MILGTALTGLFSVVAQILVPLAATLASPDKRGKVVGTIMSGLLLGILLARTVAGLLANL
GGWRTVFWVASVLMALMALALALWRGLPQMKSETIILNYPQLLGSVFSMFISDKILRTRAL
LGCLTFANFSILWTSMAFLLAAPPFNYSDGVIGLFGLAGAAGALGARPAGGFADKGKSHHT
TTFGLLLLLLSWLAIWFGHTSVLALIIGILVLDLTVQGVHITNQTVIYRIHPDARNRLTAGYM
TSYFIGGAAGSLISASAWQHGGWAGVCLAGATIALVNLLVWWRGFHRQEAAN (SEQ ID
No.6).

In this context, the ygaY gene can be any one of the following:
P1) a DNA molecule with the nucleotide sequence of SEQ ID No.7;
P2) a DNA molecule with more than 75% identity to the nucleotide sequence defined in P1);
P3) a DNA molecule that hybridizes to the nucleotide sequence defined in P1)-P2) under stringent conditions.

In a specific embodiment, the nucleotide sequence of ygaY is: 5'-

atgatcctcggtacggcattaaccggtttattctcagtcgtggcacaaattctggttccgctggcagcgacgctggcttcaccggataaacgcggc
aaagtggttggcactattatgagcggtctgctgttggggatcttgctggcacggacagttgccggattgctggcgaatctcggcggctggcgcac
cgtcttttgggttgcttcggtgttaatggcactgatggcgctggcattatggcgtggtctgccacaaatgaaatcagaaacccacctcaactaccca
cagttgttgggttccgttttcagtatgtttatcagcgataaaattctgcgtacccgcgcgttgctgggctgcctgacctttgccaatttcagcattctct
ggacctcaatggcctttttgcttgccgctccaccttttaactacagcgatggtgtcattggtctgtttggacttgcgggagctgccggagcgttggg
cgctcgtccggcgggcggttttgccgataagggcaaatcgcaccacaccacaactttcggtctgctgctgctattactttcatggctggcgatctg
gtttggtcacacttccgtactggcgttgattatcggaatcctggtgctggatctcaccgtgcagggcgtgcatatcactaaccagacggtaatttatc
ggatacatcctgatgcgcgcaatcgcctgaccgcaggttacatgactagctagctactttattggcggtgccgccggttcgctaatttcagcctca
gcctggcaacatggcggttgggctggcgtttgtctggctggcgcgacgattgccctggttaacttactggtctggtggcgaggttttcatcgtcag
gaagccgcaaattaa-3' (SEQ ID No.7).

The aforementioned recombinant Escherichia coli also comprises a promoter that initiates the transcription of the cyanate transporter or carbonic anhydrase-encoding gene.

The promoter can be a Ptrc promoter, which is any one of the following DNA molecules:
1) a DNA molecule with the nucleotide sequence of SEQ ID No.1 in one strand;
2) a DNA molecule with more than 80% identity to the DNA molecule of 1) and possessing promoter function.

In a specific embodiment, the nucleotide sequence of the Ptrc promoter is:

5'-TTGACAATTAATCATCCGGCTCGTATAATGTGTGGAATTGTGAGCGGATAACAATTTCA
CACAGGAAACAGACC-3' (SEQ ID No.1).

The aforementioned recombinant Enterobacteriaceae can be recombinant Escherichia coli, and the aforementioned Enterobacteriaceae can be Escherichia coli.

The recipient Escherichia coli can be wild-type Escherichia coli or modified Escherichia coli.

The wild-type Escherichia coli can be Escherichia coli W3110.

The modified Escherichia coli can be the genetically engineered strain E. coli W3110 (E. coli W3110 ARG10).

The recombinant Escherichia coli can be ARG-cynX and W3110-cynX, ARG-pZ8-cynX and W3110-pZ8-cynX.

The expression level of the cyanate transporter-encoding gene cynX in the recombinant Escherichia coli ARG-cynX is higher than that in the recipient Escherichia coli ARG10.

The expression level of the cyanate transporter-encoding gene cynX in the recombinant Escherichia coli W3110-cynX is higher than that in the recipient Escherichia coli W3110.

The expression level of the cyanate transporter-encoding gene in the recombinant Escherichia coli ARG-pZ8-cynX is higher than that in the recipient Escherichia coli ARG10.

The expression level of the cyanate transporter-encoding gene in the recombinant Escherichia coli W3110-pZ8-cynX is higher than that in the recipient Escherichia coli W3110.

The recombinant Escherichia coli ARG-cynX, W3110-cynX, ARG-pZ8-cynX, and W3110-pZ8-cynX do not comprise the complete coding region of the predicted transporter protein-encoding gene ygaY, which is 882 bp in length. The 215th to 405th positions of the ygaY coding region (positions 215-405 of SEQ ID No.7) are replaced by cynX initiated by Ptrc.

The recombinant Escherichia coli can also be ARG-cynT and W3110-cynT, ARG-pZ8-cynT and W3110-pZ8-cynT.

The expression level of the carbonic anhydrase-encoding gene cynT in the recombinant Escherichia coli ARG-cynT is higher than that in the recipient Escherichia coli ARG10.

The expression level of the carbonic anhydrase-encoding gene cynT in the recombinant Escherichia coli W3110-cynT is higher than that in the recipient Escherichia coli W3110.

The expression level of the carbonic anhydrase-encoding gene in the recombinant Escherichia coli ARG-pZ8-cynT is higher than that in the recipient Escherichia coli ARG10.

The expression level of the carbonic anhydrase-encoding gene in the recombinant Escherichia coli W3110-pZ8-cynT is higher than that in the recipient Escherichia coli W3110.

The recombinant Escherichia coli ARG-cynT and W3110-cynT, ARG-pZ8-cynT and W3110-pZ8-cynT do not comprise the complete coding region of the predicted transporter protein-encoding gene ygaY, which is 882 bp in length. The 215th to 405th positions of the ygaY coding region (positions 215-405 of SEQ ID No.7) are replaced by cynT initiated by Ptrc.

The vector mentioned herein refers to a carrier capable of transporting exogenous DNA or target genes into host cells for amplification and expression. The vector can be a cloning vector or an expression vector, including but not limited to plasmids, phages (such as λ phage or M13 filamentous phage), cosmids (i.e., cosmid plasmids), or viral vectors.

The microorganisms mentioned herein can be yeasts, bacteria, algae, or fungi. Among them, bacteria can be from Escherichia sp., Erwinia sp., Agrobacterium sp., Flavobacterium sp., Alcaligenes sp., Pseudomonas sp., Bacillus sp., Brevibacterium sp., Corynebacterium sp., Aerobacter sp., Enterobacteria sp., Micrococcus sp., Serratia sp., Salmonella sp., Streptomyces sp., Providencia sp., etc., but are not limited thereto.

Furthermore, the bacteria can be Escherichia coli, Corynebacterium glutamicum, Brevibacterium lactofermentum, Brevibacterium flavum, or Corynebacterium pekinense.

The cells mentioned herein can be plant cells or animal cells. The cells can be any biological cells capable of synthesizing the target amino acid.

The present disclosure also provides a method for constructing the aforementioned recombinant Escherichia coli.

The present disclosure provides a method for constructing the aforementioned recombinant Escherichia coli, which is method A or method B:
Method A involves regulating the arginine production of microorganisms by regulating the expression of the encoding gene of the aforementioned cyanate transporter or regulating the activity or content of the cyanate transporter, resulting in microorganisms with altered arginine production;
Method B involves regulating the arginine production of microorganisms by regulating the expression of the encoding gene of the aforementioned carbonic anhydrase or regulating the activity or content of the carbonic anhydrase, resulting in microorganisms with altered arginine production.

By regulating the expression of the encoding gene of the proteins in the L-arginine metabolic pathway mentioned above or regulating the activity or content of the proteins in the L-arginine metabolic pathway, the arginine production of microorganisms can be regulated, resulting in microorganisms with altered arginine production.

In the aforementioned methods, the method for regulating the expression of the encoding gene of the cyanate transporter or regulating the activity or content of the cyanate transporter can be any one of the following:
M1) introducing the encoding gene of the cyanate transporter into the target microorganisms;
M2) introducing the encoding gene with the amino acid sequence of SEQ ID No.4 into the target microorganisms;
in the aforementioned methods, the method for regulating the expression of the encoding gene of the carbonic anhydrase or regulating the activity or content of the carbonic anhydrase is any one of the following:
N1) introducing the encoding gene of the carbonic anhydrase into the target microorganisms;
N2) introducing the encoding gene with the amino acid sequence of SEQ ID No.5 into the target microorganisms.

In an embodiment, the aforementioned recombinant Escherichia coli is constructed by a method comprising the following steps: replacing positions 215-405 of the nucleotide sequence SEQ ID No.7 of the ygaY gene of the recipient Escherichia coli with the encoding gene of the cyanate transporter or carbonic anhydrase.

The ygaY gene encodes the predicted transporter protein YgaY.

In the aforementioned methods, the encoding gene of the cyanate transporter can be any one of the following:
C1) a DNA molecule with the nucleotide sequence of SEQ ID No.2;
C2) a DNA molecule with 75% or more identity to the nucleotide sequence defined in C1) and encoding the cyanate transporter;
C3) a DNA molecule that hybridizes to the nucleotide sequence defined in C1)-C2) under stringent conditions and encodes the cyanate transporter.

In the aforementioned methods, the encoding gene of the carbonic anhydrase can be any one of the following:
D1) a DNA molecule with the nucleotide sequence of SEQ ID No.3;
D2) a DNA molecule with more than 75% identity to the nucleotide sequence defined in D1) and encoding the carbonic anhydrase;
D3) a DNA molecule that hybridizes to the nucleotide sequence defined in D1)-D2) under stringent conditions and encodes the carbonic anhydrase.

In the aforementioned methods, the predicted transporter ygaY gene encodes any one of the following proteins:
H1) a protein encoded by the predicted transporter-encoding gene ygaY with an amino acid sequence of SEQ ID No.6;
H2) a protein obtained by substituting and/or deleting and/or adding amino acid residues from the amino acid sequence of H1), which has more than 80% identity to the protein of H1);
H3) a fusion protein obtained by linking a tag to the N-terminus and/or C-terminus of H1) or H2), which possesses predicted transporter protein activity.

The present disclosure also provides the uses of the aforementioned strains in any one of the following:
E1) regulating the arginine production of microorganisms;
E2) preparing arginine;
E3) constructing genetically engineered microorganisms for arginine production.

The present disclosure also provides the uses of the aforementioned strains in producing arginine or foods, pharmaceuticals, and/or feeds comprising arginine.

The present disclosure also provides the uses of proteins in any one of the following:
F1) regulating the arginine production of microorganisms;
F2) preparing arginine;
F3) constructing genetically engineered microorganisms for arginine production.

The proteins can be protein A or protein B, where protein A is a cyanate transporter with the amino acid sequence of SEQ ID No.4, and protein B is a carbonic anhydrase with the amino acid sequence of SEQ ID No.5.

The present disclosure also provides the uses of the aforementioned construction methods in any one of the following:
E1) regulating the arginine production of microorganisms;
E2) preparing arginine;
E3) constructing genetically engineered microorganisms for arginine production.

The present disclosure also provides the uses of the aforementioned construction methods in producing arginine or foods, pharmaceuticals, and/or feeds comprising arginine.

The present disclosure also provides nucleic acid molecules, which are nucleic acid molecule A or nucleic acid molecule B; nucleic acid molecule A encodes the cyanate transporter in the aforementioned Enterobacteriaceae; nucleic acid molecule B encodes the carbonic anhydrase in the aforementioned Enterobacteriaceae.

The present disclosure also provides expression cassettes, which are expression cassette A or expression cassette B, where expression cassette A comprises the aforementioned nucleic acid molecule A, and expression cassette B comprises the aforementioned nucleic acid molecule B.

The present disclosure also provides recombinant vectors, which are recombinant vector A or recombinant vector B, where recombinant vector A comprises the aforementioned nucleic acid molecule A or the aforementioned expression cassette, and recombinant vector B comprises the aforementioned nucleic acid molecule B or the aforementioned expression cassette.

The present disclosure also provides whole-cell catalysts, which are whole-cell catalyst A or whole-cell catalyst B, where whole-cell catalyst A comprises the aforementioned nucleic acid molecule A, and whole-cell catalyst B comprises the aforementioned nucleic acid molecule B.

The present disclosure also provides a method for preparing L-arginine, which comprises culturing the aforementioned recombinant Escherichia coli to obtain a fermentation product and obtaining L-arginine from the fermentation product.

The recombinant microorganisms or recombinant cells constructed by overexpressing cynX or cynT using the present disclosure can also be used to produce various products, including but not limited to lysine, glutamic acid, glycine, alanine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutaric acid, citric acid, ornithine, and/or citrulline.

### DETAILED DESCRIPTION

The present disclosure will be further described in detail below in conjunction with specific embodiments. The provided examples are solely intended to elucidate the disclosure and not to limit its scope. The examples presented hereinafter can serve as a guide for further improvements by professionals in the technical field and do not in any way constitute a restriction on the disclosure.

Unless otherwise specified, the experimental methods in the following examples are conventional methods and are carried out in accordance with the techniques or conditions described in the literature within the field or according to the product instructions. Unless otherwise specified, the materials, reagents, etc., used in the following examples can be obtained through commercial channels.

Unless otherwise specified, for the quantitative experiments in the following examples, three replicate experiments are set up, and the results are taken as the average value.

The pREDCas9 plasmid in the following examples has been documented in: Jiang W, Bikard D, Cox D, Zhang F, Marrafni LA (2013) RNA-guided editing of bacterial genomes using CRISPR-Cas systems. Nat Biotechnol 31:233 - 239. https://doi.org/10.1038/nbt.2508. The public can obtain this biological material from the applicant. This biological material is solely for the purpose of repeating the experiments of the present disclosure and cannot be used for other purposes.

The shuttle expression vector pZ8 in the following examples has been documented in: Huang Qinqin, Wang Huimei, Liang Ling, Huang Qingeng, Wu Songgang, et al. Effects of lysC site-directed mutagenesis and tandem expression of lysC and asdA on L-threonine accumulation in Corynebacterium glutamicum. Biotechnology Bulletin [J]. 2019(035)002. The public can obtain this biological material from the applicant. This biological material is solely for the purpose of repeating the experiments of the present disclosure and cannot be used for other purposes.

The modified Escherichia coli E. coli W3110 ARG10 in the following examples, also known as the genetically engineered bacterium E. coli W3110 ARG10, is constructed according to the method in Example 1 of CN 110964683 B.

The wild-type strain Escherichia coli W3110 in the following examples is purchased from Bio-Lab with the product number BTN12-170201y.

In the following examples, SPSS11.5 statistical software is used for data processing. The experimental results are expressed as the mean ± standard deviation. One-way ANOVA is employed for testing. P < 0.05 (*) indicates a significant difference, P < 0.01 (**) indicates a highly significant difference, and P < 0.001 (***) indicates an extremely significant difference.

In the following examples, primers are designed according to the principle of homologous recombination. Primer synthesis and sequencing are completed by Genewiz (Suzhou) Biotechnology Co., Ltd. The specific sequences are detailed in Table 1 below.

**Table 1. Primers used in the present disclosure**

| Primers | Sequence (5'-3') | Position in the sequence listing | Purpose (amplification/knockout /validation) |
|---|---|---|---|
| UP-*ygaY*-S | CCTACAAACCACATCGCACATT | SEQ ID No.12 | Amplify the upstream homologous arm of |
| UP*-ygaY*-A | AATTGTTATCCGCTCACAATTCCAC ACATTATACGAGCCGGATGATTAAT TGTCAAACACCGAAGCAACCCAA AAG | SEQ ID No. 13 | the cynT gene for integration at the ygaY locus |
| *cynT-*S | TCCGGCTCGTATAATGTGTGGAATT GTGAGCGGATAACAATTTCACACA GGAAACAGACCGTGAAAGAGATTA TTGATGGATTCC | SEQ ID No.14 | Amplify the cynT gene integrated at the ygaY locus |
| *cynT-*A | GGTGGAGCGGCAAGCAATTACGCT GCGGTCGGTTGG | SEQ ID No.15 | |
| DN*-ygaY-*S | CCAACCGACCGCAGCGTAATTGCT TGCCGCTCCACC | SEQ ID No.16 | Amplify the downstream homologous arm of the cynT gene for integration at the ygaY locus |
| DN*-ygaY-*A | GGAGTAGGGCTTTCCATAGAGTGT | SEQ ID No.17 | |
| gRNA-*ygaY-*S | AGTCCTAGGTATAATACTAGTCTCA ACTACCCACAGTTGTTGTTTTAGA GCTAGAA | SEQ ID No.18 | Amplify the ygaY sgRNA sequence |
| *gRNA-ygaY-*A | TTCTAGCTCTAAAACAACAACTGT GGGTAGTTGAGACTAGTATTATACC TAGGACT | SEQ ID No.19 | |
| *cynT-*pS | TGAGCGGATAACAATTTCACACAG GAAACAGAATTCGTGAAAGAGATT ATTGATGGATTCC | SEQ ID No.20 | Amplify the cynT gene for expression in the pZ8 vector |
| *cynT-*pA | TCTCATCCGCCAAAACAGAAGCTT GGCTGCAGGTCGACTTACGCTGCG GTCGGTT | SEQ ID No.21 | |
| pZ8F | AGCGGATAACAATTTCACACAGG | SEQ ID No.22 | Identification primers for the pZ8-cynT vector |
| pZ8R | CAGGCTGAAAATCTTCTCTC | SEQ ID No.23 | |
| UP*-cynT-*S | AATCCCAACGTCCAACTCG | SEQ ID No.24 | Amplify the upstream homologous arm for cynT knockout |
| UP-*cynT-*A | ACTCCTGATGGTTTAAAAATAAGG CGTTAACCTCTGTCTGTCTC | SEQ ID No.25 | |
| DN*-cynT-*S | GAGACAGACAGAGGTTAACGCCTT ATTTTTAAACCATCAGGAGT | SEQ ID No.26 | Amplify the downstream |
| DN-*cynT-*A | GATGGTTGCGCATGGCTG | SEQ ID No.27 | homologous arm for cynT knockout |
| gRNA*-cynT-*S | AGTCCTAGGTATAATACTAGTATTT GTGGTCATTCCAACTGGTTTTAGA GCTAGAA | SEQ ID No.28 | Primers for amplifying the cynT sgRNA sequence |
| gRNA*-cynT-*A | TTCTAGCTCTAAAACCAGTTGGAA TGACCACAAATACTAGTATTATACC TAGGACT | SEQ ID No.29 | |
| YPpGRBF | CTAGTATTATACCTAGGACTGAGC | SEQ ID No.30 | Amplify the linearized pGRB vector |
| YPpGRBR | GTTTTAGAGCTAGAAATAGCAAGT | SEQ ID No.31 | |
| pCasF | GCAGTGGCGGTTTTCATGGC | SEQ ID No.32 | Determine whether the pREDCas9 vector has been introduced into the host strain |
| pCasR | CCTTGGTGATCTCGCCTTTCACG | SEQ ID No.33 | |
| *cynX-*S | TCCGGCTCGTATAATGTGTGGAATT GTGAGCGGATAACAATTTCACACA GGAAACAGACCATGCTGCTGGTAC TGGTGCT | SEQ ID No.34 | Amplify the cynX gene integrated at the ygaY locus |
| *cynX-*A | GGTGGAGCGGCAAGCAATCATGCC TCTTTGACCCAC | SEQ ID No.35 | |
| DN*-ygaY-*S | GTGGGTCAAAGAGGCATGATTGCT TGCCGCTCCACC | SEQ ID No.36 | Amplify the downstream homologous arm of the cynX gene integrated at the ygaY locus |
| DN*-ygaY-*A | GGAGTAGGGCTTTCCATAGAGTGT | SEQ ID No.37 | |
| *cynX-*pS | TGAGCGGATAACAATTTCACACAG GAAACAGAATTCATGCTGCTGGTA CTGGTG | SEQ ID No.38 | Amplify the cynX gene for expression in the pZ8 vector |
| *cynX-*pA | TCTCATCCGCCAAAACAGAAGCTT GGCTGCAGGTCGACTCATGCCTCT TTGACCCAC | SEQ ID No.39 | |
| UP*-cynX-*S | GTTTGTGCCATACCGCTACG | SEQ ID No.40 | Amplify the upstream homologous arm for cynX knockout |
| UP*-cynX-*A | CGCAGGAACAAGCGTCGCACGTG AACGTCTTTTGATGG | SEQ ID No.41 | |
| DN*-cynX-*S | CCATCAAAAGACGTTCACGTGCGA CGCTTGTTCCTGCG | SEQ ID No.42 | Amplify the downstream homologous arm for cynX knockout |
| DN*-cynX-*A | GGGTAGAACCGCCTGTCTAT | SEQ ID No.43 | |
| gRNA*-cynX-*S | AGTCCTAGGTATAATACTAGTACTG CTACCGCAATTGCGCCGTTTTAGA GCTAGAA | SEQ ID No.44 | For amplification of the cynX sgRNA sequence |
| gRNA*-cynX-*A | TTCTAGCTCTAAAACGGCGCAATT GCGGTAGCAGTACTAGTATTATACC TAGGACT | SEQ ID No.45 | |

| | | | |
|---|---|---|---|
| Note: The underlined nucleotide sequences are homologous sequences. | | | |

The specific information of the strains used in this disclosure is shown in Table 2.

**Table 2. Strains and plasmids used in the present disclosure**

| Name of the strain | Information Description | Source |
|---|---|---|
| ARG10 | For details, refer to Chinese Patent No. CN 110964683 B. | Constructed by our laboratory |
| ARG-cynX | Overexpress the cynX gene in the genome of the ARG10 strain | The present disclosure |
| ARG-pZ8-cynX | Overexpress the cynX gene on the pZ8 vector in the ARG10 strain | The present disclosure |
| ARG:ΔcynX | Knock out the cynX gene in the ARG10 strain | The present disclosure |
| W3110 | Escherichia coli model strain | Bio-Lab |
| W3110-cynX | Overexpress the cynX gene in the genome of W3110. | The present disclosure |
| W3110-pZ8-cynX | Overexpress the cynX gene on the pZ8 vector in the W3110 strain. | The present disclosure |
| W3110:ΔcynX | Knock out the cynX gene in the W3110 strain. | The present disclosure |
| ARG-cynT | Overexpress cynT in ARG10 | The present disclosure |
| ARG-pZ8-cynT | Overexpress the cynT gene on the pZ8 vector in the ARG10 strain | The present disclosure |
| ARG:ΔcynT | Knock out the cynT gene in the ARG10 strain | The present disclosure |
| W3110-cynT | Overexpress the cynT gene in the genome of the W3110 strain | The present disclosure |
| W3110-pZ8-cynT | Overexpress the cynT gene on the pZ8 vector in the W3110 strain | The present disclosure |
| W3110:ΔcynT | Knock out the cynT gene in the W3110 strain | The present disclosure |

In the following examples, the composition of the slope culture medium is as follows: glucose 1-5 g/L, peptone 5-10 g/L, beef extract 5-10 g/L, yeast extract 1-5 g/L, NaCl 1-2.5 g/L, agar 15-20 g/L, with the remainder being water, and the pH is 7.0-7.2.

In the following examples, the composition of the seed culture medium is as follows: glucose 20-40 g/L, yeast extract 2-5 g/L, peptone 2-4 g/L, K₂HPO₄ 1-3 g/L, MgSO₄·7H₂O 1-2 g/L, FeSO₄·7H₂O 15-20 mg/L, MnSO₄·7H₂O 15-20 mg/L, and each of V_{B1}, V_{B3}, V_{B5}, V_{B12}, and V_{H} at 1-3 mg/L, with the remainder being water, and the pH is 7.0-7.2.

In the following examples, the composition of the fermentation medium is as follows: glucose 20-40 g/L, yeast extract 1-3 g/L, peptone 2-3 g/L, K₂HPO₄ 3-6 g/L, MgSO₄·7H₂O 1-2 g/L, FeSO₄·7H₂O 15-20 mg/L, MnSO₄·7H₂O 15-20 mg/L, and each of V_{B1}, V_{B3}, V_{B5}, V_{B12}, and V_{H} at 1-3 mg/L, with the remainder being water, and the pH is 7.0-7.2.

Unless otherwise specified, the experimental methods in the following examples are conventional methods and are carried out in accordance with the techniques or conditions described in the literature within the field or according to the product instructions.

The detailed steps of some experimental methods in the following examples are as follows:
I. Gene Editing Method: This is carried out with reference to the literature (Li Y, Lin Z, Huang C, et al. Metabolic engineering of Escherichia coli using CRISPR-Cas9 mediated genome editing. Metabolic engineering, 2015, 31: 13-21.). Here, pREDCas9 carries an elimination system for the gRNA expression plasmid pGRB, the Red recombination system of λ phage, and the Cas9 protein expression system. It exhibits spectinomycin resistance (working concentration: 100 mg/L) and is cultured at 32°C. pGRB includes the promoter J23100, the gRNA-Cas9 binding region sequence, and the terminator sequence, exhibiting ampicillin resistance (working concentration: 100 mg/L) and cultured at 37°C.

The specific steps for constructing the pGRB plasmid in this disclosure are as follows:

### I.Construction of pGRB Plasmid

The purpose of constructing the plasmid pGRB is to transcribe the corresponding gRNA, which forms a complex with the Cas9 protein and recognizes the target site of the gene of interest through base pairing and PAM recognition, achieving double-strand breaks in the target DNA. The pGRB plasmid is constructed using a method involving recombination between a DNA fragment comprising the target sequence and a linearized vector fragment.

### 1.1 Design of Target Sequence

Design the target sequence using CRISPR RGEN Tools (PAM: 5'-NGG-3').

### 1.2 Preparation of DNA Fragment Comprising Target Sequence

Design primers: 5'-linearized vector end sequence (15 bp)-restriction enzyme site-target sequence (excluding PAM sequence)-linearized vector end sequence (15 bp)-3' and its reverse complementary primer. The DNA fragment comprising the target sequence is prepared by annealing single-stranded DNA. The specific target sequences in this disclosure are as follows: 5'-ctcaactacccacagttgtt-3' (for knocking out the ygaY gene, SEQ ID No.46), 5'-Atttgtggtcattccaactg-3' (for knocking out the cynT gene, SEQ ID No.47), and 5'-ACTGCTACCGCAATTGCGCC-3' (for knocking out the cynX gene, SEQ ID No.48).

Reaction conditions: Pre-denaturation at 95°C for 5 min; annealing at 30-50°C for 1 min. The 20 µL annealing system is as follows:

**Table 3. 20 µL Annealing System**

| Reaction system | Volume(µL) |
|---|---|
| Primers (10 µmol/L) | 10 |
| Reverse Complementary Primer (10 µmol/L) | 10 |

### 1.3 Preparation of Linearized Vector

The vector is linearized using the inverse PCR amplification method. The plasmid pGRB is amplified with primers YPpGRBF and YPpGRBR.

### 1.4 Recombination Reaction

The recombination system is shown in Table 4 below. All recombination enzymes used are from the ClonExpress ^{®} II One Step Cloning Kit series. Recombination conditions: 37°C for 30 min.

**Table 4. 20 µL Recombination System**

| Reaction system | Volume (µL) |
|---|---|
| 5 × CE II Buffer | 4 |
| Linearized cloning vector | 1 |
| Target Sequence Fragment | 1 |
| Exnase^{®} II | 2 |
| ddH₂O | 12 |

### 1.5 Transformation of Plasmid

Take 10 µL of the recombination reaction solution from step 1.4 and add it to 100 mL of DH5α chemically competent cells. Gently mix and then incubate on ice for 20 min. Heat shock at 42°C for 45-90 s, followed by immediate incubation on ice for 2-3 min. Add 900 µL of SOC medium and recover at 37°C for 1 h. Centrifuge at 8000 rpm for 2 min, discard part of the supernatant, leaving approximately 200 µL to resuspend the cells, and then spread onto plates comprising 100 mg/L ampicillin. Invert the plates and incubate overnight at 37°C. After single colonies appear on the plates, identify them by colony PCR and select positive recombinants.

### 1.6 Clone Identification

Inoculate PCR-positive colonies into LB medium comprising 100 mg/L ampicillin and culture overnight. Preserve the bacteria, extract the plasmid, and perform restriction enzyme digestion identification to obtain the recombinant plasmid pGRB-sgRNA.

The structural description of the recombinant plasmid pGRB-sgRNA is as follows: the small fragment between the homologous arms 5'-gctcagtcctaggtataatactagt-3' (SEQ ID No.49) and 5'-gttttagagctagaaatagcaagt-3' (SEQ ID No.31) of the pGRB vector is replaced with a DNA fragment comprising the target sequence 5'-ctcaactacccacagttgtt-3' (for knocking out the ygaY gene, SEQ ID No.46) or 5'-Atttgtggtcattccaactg-3' (for knocking out the cynT gene, SEQ ID No.47) or 5'-ACTGCTACCGCAATTGCGCC-3' (for knocking out the cynX gene, SEQ ID No.48), while keeping the other sequences of the pGRB vector unchanged, resulting in the recombinant expression vector.

### II. Transformation of the pREDCas9 Vector and Preparation of Competent Cells of the Target Strain Harboring pREDCas9

### 1.Electrotransformation of pREDCas9

The pREDCas9 plasmid is electrotransformed into the electrocompetent cells of the starting strain using an electroporation method. After recovery and culture, the cells are spread onto LB plates comprising spectinomycin (at a concentration of 100 mg/L) and incubated overnight at 32°C. Single colonies growing on the resistant plates are subjected to colony PCR using the identification primer set pCasF/pCasR (specific sequences are shown in Table 1) to screen for positive recombinants.

### 2.Preparation of Competent Cells of the Target Strain Harboring pREDCas9

When the target strain harboring pREDCas9 is cultured at 32°C to an OD₆₀₀ of 0.1 to 0.2, 0.1 M IPTG is added (to achieve a final concentration of 0.1 mM), and the culture is continued until an OD₆₀₀ of 0.6 to 0.7 is reached, at which point competent cells are prepared. The purpose of adding IPTG is to induce the expression of recombinase on the pREDCas9 plasmid. The medium required for competent cell preparation and the preparation process follow standard routine operating procedures.

### Example 1: Application of the cynX Protein and Its Encoding Gene in Regulating Arginine Production

### A. Construction of the Genetic Engineering Strain ARG-cynX

### 1.Preparation of Recombinant DNA Fragments

The recombinant fragment for cynX gene overexpression is composed of upstream and downstream homologous arms of the cynX gene (ygaY::P_{trc}-cynX). Using the primer design software Primer5, primers for the upstream and downstream homologous arms (with an amplification length of approximately 400-800 bp) are designed based on the upstream and downstream sequences of the cynX gene. The upstream and downstream primers for the upstream homologous arm are UP-ygaY-S/UP-ygaY-A (specific sequences are shown in Table 1); the upstream and downstream primers for cynX are cynX-S/cynX-A (specific sequences are shown in Table 1); the upstream and downstream primers for the downstream homologous arm are DN-ygaY-S/DN-ygaY-A (specific sequences are shown in Table 1). After separately amplifying the upstream and downstream homologous arms and the target gene fragment by PCR, the recombinant fragment ygaY::P_{trc}-cynX is prepared by overlap PCR. The nucleotide sequence of the recombinant fragment ygaY::P_{trc}-cynX is Sequence 8 in the sequence listing.

The 50 µL PCR system and method are shown in Table 5 below.

**Table 5. PCR Amplification System**

| Component | Volume(µL) |
|---|---|
| DNA template | 1 |
| The upstream primers (10 µmol/L) | 1 |
| The downstream primers (10 µmol/L) | 1 |
| dNTP mixture(10 mmol/L) | 4 |
| 5 × Buffer | 10 |
| HS enzyme (5 U/µL) | 0.5 |
| ddH₂O | 32.5 |

The 50 µL overlap PCR system is shown in Table 6 below.

**Table 6. Overlap PCR Amplification System**

| Component | Volume(µL) |
|---|---|
| Template | 2 |
| The upstream primers for the upstream homologous arm(10 µmol/L) | 1 |
| The downstream primers for the downstream homologous arm(10 µmol/L) | 1 |
| dNTP mixture(10 mmol/L) | 4 |
| 5 × Buffer | 10 |
| HS enzyme (5 U/µL) | 0.5 |
| ddH₂O | 31.5 |

| | |
|---|---|
| Note: The template is composed of equimolar amounts of the amplified fragments of the upstream and downstream homologous arms and the target gene, with a total amount not exceeding 10 ng. | |

PCR reaction conditions (using Takara PrimeSTAR HS enzyme): pre-denaturation at 95°C for 5 min; followed by 30 cycles of denaturation at 98°C for 10 s, annealing at ((Tm-3/5)°C) for 15 s, and extension at 72°C (this enzyme extends approximately 1 kb per minute); a final extension at 72°C for 10 min; and maintenance at 4°C.

### 2.Preparation of Electrocompetent Cells Harboring pREDCas9

For the specific method of preparing electrocompetent cells harboring pREDCas9, Experimental Method II described earlier is referred to.

### 3.Transformation of pGRB and Recombinant DNA Fragments

The pGRB prepared in Experimental Method I described earlier and the recombinant DNA fragment obtained in Step 1 of Part A of this example are simultaneously electrotransformed into the electrocompetent cells harboring pREDCas9 from Step 2. After recovery and culture following electroporation, the cells are spread onto LB plates comprising ampicillin (at a concentration of 100 mg/L) and spectinomycin (at a concentration of 100 mg/L) and incubated overnight at 32°C. Colony PCR verification is performed using the specifically designed identification primer set UP-ygaY-S and DN-ygaY-A (specific sequences are shown in Table 1). Strains with a 2462 bp fragment are identified as positive strains, and the selected positive recombinants are preserved.

### 4.Elimination of Plasmids

### 1) Elimination of pGRB

The positive recombinants obtained in Step 3 are cultured overnight in LB medium comprising 0.2% arabinose, diluted appropriately, and then spread onto LB plates comprising spectinomycin resistance and incubated overnight at 32°C. The plates are then spotted with LB plates comprising both ampicillin and spectinomycin resistance. Single colonies that grew on the spectinomycin-resistant plate but not on the ampicillin plate are selected and preserved.

### 2) Elimination of the pREDCas9 plasmid

The positive recombinants are transferred into antibiotic-free LB liquid medium and cultured overnight at 42°C. After appropriate dilution, they are spread onto antibiotic-free LB plates and incubated overnight at 37°C. The plates are then spotted with LB plates comprising spectinomycin resistance and antibiotic-free LB plates. Single colonies that grew on the antibiotic-free plate but not on the spectinomycin-resistant plate are selected and preserved.

### 5.ygaY::P_{trc}-cynX Gene Editing

1) the genome of Escherichia coli W3110 is used as a template, upstream homologous arm primers (UP-ygaY-S, UP-ygaY-A) and downstream homologous arm primers (DN-ygaY-S, DN-ygaY-A) are designed based on the upstream and downstream sequences of the ygaY gene (NCBI GeneID: 2847696), obtaining the upstream homologous arm fragment UP-ygaY and the downstream homologous arm fragment DN-ygaY.
2) the genome of Escherichia coli W3110 is used as a template, primers cynX-S and cynX-A are designed based on the upstream and downstream sequences of the cynX gene (NCBI GeneID: 948767) and the Ptrc promoter sequence to amplify the cynX gene, obtaining the recombinant fragment Ptrc-cynX.
3) The upstream homologous arm fragment UP-ygaY, downstream homologous arm fragment DN-ygaY, and recombinant fragment Ptrc-cynX are fused by overlap PCR to obtain ygaY::P_{trc}-cynX (upstream homologous arm-Ptrc-cynX-downstream homologous arm). The upstream homologous arm should be 616 bp in length, the Ptrc promoter 74 bp, the cynX fragment 1155 bp, the downstream homologous arm 617 bp, and the total length of the overlapping fragment should be 2462 bp. The nucleotide sequence of the overlapping fragment is SEQ ID No.8 in the sequence listing.

The nucleotide sequence of the overlapping fragment is:

5’-cctacaaaccacatcgcacatttcaacaatctatctttcatcccatattcatcaacatccgctattattgatttccagcttaatcatcacctgatgaa

caaaaataatgactaaacctaatcatgagcttagcccggcgctgatcgtgctgatgtctatcgccaccggtctggcggtagccagtaactattacg

cccagccattgctcgacaccatcgcgcgtaacttttccctttccgccagttcggcaggctttattgttaccgccgcgcagttgggctatgccgcag

gtctactgtttcttgttcccctcggtgatatgtttgaacgccgccgcctgattgtctcgatgaccttactggcggcatgttgattaccgccagcagtca

gtcgctggcgatgatgatcctcggtacggcattaaccggtttattctcagtcgtggcacaaattctggttccgctggcagcgacgctggcttcacc

ggataaacgcggcaaagtggttggcactattatgagcggtctgctgttggggatcttgctggcacggacagttgccggattgctggcgaatctcg

gcggctggcgcaccgtcttttgggttgcttcggtgtttgacaattaatcatccggctcgtataatgtgtggaattgtgagcggataacaatttcacac
aggaaacagaccatgctgctggtactggtgctgattggtcttaatatgcgaccactgctcacctccgtcgggccactgctaccgcaattgcgcca
ggcgagcggaatgagctttagcgtggctgccctgttgaccgctctgccggtggttaccatgggcgggctggcgctggccggaagctggcttca
tcagcatgtcagcgaacgtcgcagtgtcgccatcagtctgttgctgattgccgtcggtgcattgatgcgtgagctttacccgcaaagtgcgctgct
gcttagcagcgcactgcttggtggggtggggatcggcatcattcaggcggtgatgccttcggtgattaaacggcggtttcagcagcgcacgcca
ctggtgatggggctgtggtccgcggctctgatgggcggcggtgggcttggtgccgccataacgccctggttagttcaacatagcgaaacctggt
atcaaacactcgcctggtgggcgctgcctgccgttgttgcgctctttgcctggtggtggcaaagcgcccgcgaggtcgcctcttcccacaagac
aacaaccactccggttcgcgtggtattcactccccgcgcgtggacgctgggtgtttacttcggtctgattaacggcggttacgccagcctgattgc
ctggttacccgctttctatattgagattggtgccagcgcgcagtacagcggttccttactggcattgatgacgcttgggcaagccgcaggagcttt
gctgatgcctgctatggctcgccatcaggatcggcgcaaactgttaatgctggcgctggtgttacaactggtggggttctgcggctttatctggct
gccgatgcaattgccggtattgtgggcgatggtgtgtgggttaggtctgggcggcgcgtttccgctctgtttgctgctggcgctcgatcactctgtg
caaccggctattgctggcaagctggtggcgtttatgcagggaatcggttttatcatcgccgggcttgccccgtggttttctggcgtgctgcgtagta
tcagcggcaattacctgatggactgggcatttcatgcgctgtgcgtcgttgggctgatgatcataaccctgcgttttgcaccagtacgttttccgca
gctgtgggtcaaagaggcatgattgcttgccgctccaccttttaactacagcgatggtgtcattggtctgtttggacttgcgggagctgccggagc
gttgggcgctcgtccggcgggcggttttgccgataagggcaaatcgcaccacaccacaactttcggtctgctgctgctattactttcatggctggc
gatctggtttggtcacacttccgtactggcgttgattatcggaatcctggtgctggatctcaccgtgcagggcgtgcatatcactaaccagacggta
atttatcggatacatcctgatgcgcgcaatcgcctgaccgcaggttacatgactagctactttattggcggtgccgccggttcgctaatttcagcctc
agcctggcaacatggcggttgggctggcgtttgtctggctggcgcgacgattgccctggttaacttactggtctggtggcgaggttttcatcgtca
ggaagccgcaaattaagcaatcagcaaccgttttcgtggctttacacttataagggtgttaagaagcccatcagtctgataaggttaagatattcatt
cagtctatttataatattaacaatcgttaagcgtacactctatggaaagccctactcc-3’ (SEQ ID No.8).

4) Construction of pGRB-ygaY: a DNA fragment comprising the target sequence, prepared by annealing primers gRNA-ygaY-S and gRNA-ygaY-A, is ligated with the plasmid pGRB to construct the recombinant plasmid pGRB-ygaY.

The structural description of the recombinant plasmid pGRB-ygaY is as follows: it is a recombinant plasmid obtained by replacing the fragment between 5'-gctcagtcctaggtataatactagt-3' (SEQ ID No.49) and 5'-gttttagagctagaaatagcaagt-3' (SEQ ID No.31) of pGRB with the DNA molecule with the nucleotide sequence 5'-ctcaactacccacagttgtt-3' (SEQ ID No.46), while keeping the other nucleotides of pGRB unchanged.

5) competent cells of the engineering strain ARG10 (derived from patent CN 110964683 B) and the wild-type strain W3110 are prepared, the pREDCas9 plasmid is introduced, and then the target strains harboring pREDCas9 are cultured at 32°C until an OD₆₀₀ of 0.1 to 0.2 is reached. 0.1 M IPTG is added (to achieve a final concentration of 0.1 mM) and culturing is continued until an OD₆₀₀ of 0.6 to 0.7 is reached for competent cell preparation. The purpose of adding IPTG is to induce the expression of recombinase on the pREDCas9 plasmid. The medium required for competent cell preparation and the preparation process follow conventional standard operations. Experimental Method II described earlier is refer specifically to.

Colony PCR verification is performed using the primer set UP-ygaY-S and DN-ygaY-A (specific sequences are shown in Table 1). Strains with a 2462 bp fragment are identified as positive strains, while those with a 1424 bp fragment are identified as the original starting strains ARG10 and wild-type strain W3110 that has not been successfully integrated. The nucleotide sequence obtained by sequencing the PCR products of colony PCR using UP-ygaY-S and DN-ygaY-A is SEQ ID No.8 in the sequence listing.

Engineering strains ARG-cynX and W3110-cynX with enhanced cynX gene expression intensity are constructed sequentially. The first step is to construct the pGRB-cynX vector; the second step is to amplify the ygaY::P_{trc}-cynX integration fragment by PCR; the third step is to prepare competent cells of ARG10 and W3110 and transform them with pREDCas9; the fourth step is to prepare competent cells of the target strains ARG10 and W3110 harboring pREDCas9 and transform them with the pGRB-cynX vector and the ygaY::P_{trc}-cynX integration fragment; the fifth step is to eliminate the pGRB-cynX vector from the strains ARG-cynX and W3110-cynX integrated with ygaY::P_{trc}-cynX since they contained the pGRB-cynX vector; the sixth step is to eliminate the pREDCas9 vector from the target strains ARG-cynX and W3110-cynX after eliminating the pGRB-cynX vector, obtaining engineering strains ARG-cynX and W3110-cynX that do not contain the pGRB-cynX and pREDCas9 vectors and have enhanced cynX gene expression intensity. Specific steps are shown in Steps 1 to 5 of this example.

The expression level of the cyanate transporter-encoding gene cynX in the engineering strain ARG-cynX is higher than that in the recipient Escherichia coli ARG10. The expression level of the cyanate transporter-encoding gene cynX in the engineering strain W3110-cynX is higher than that in the recipient Escherichia coli W3110.

### B. Construction of genetically engineered strains ARG-pZ8-cynX and W3110-pZ8-cynX

The low-copy recombinant expression vector pZ8-cynX is constructed using the shuttle expression vector pZ8. It is then introduced into the engineered strain ARG10 and the wild-type strain W3110. The tac promoter on the pZ8 vector is used to initiate the expression of cynX to improve the yield of L-arginine.

### 1.Construction of the recombinant expression vector pZ8-cynX

Primers cynX-pS and cynX-pA (specific sequences are shown in Table 1) required for the amplification of the cynX gene are designed based on the upstream and downstream sequences of the cynX gene (NCBI GeneID: 948767). Using the Escherichia coli W3110 genome as a template, the cynX gene is amplified by PCR with the primers cynX-pS and cynX-pA, and then purified and recovered. The nucleotide sequence of the obtained cynX fragment is SEQ ID No. 2 in the sequence listing. For the PCR system and program, Step I in the experimental methods described earlier is referred to.

After the pZ8 vector is digested with EcoR I and Sal I and purified, the above-mentioned cynX PCR-purified product is recombined with the linearized pZ8 vector to obtain the recombinant vector pZ8-cynX of cynX and pZ8.

The structural description of the recombinant vector pZ8-cynX is as follows: it is a recombinant plasmid obtained by replacing the fragment between 5'-tgagcggataacaatttcacacaggaaacagaattc-3' (SEQ ID No. 50) and 5'-tctcatccgccaaaacagaagcttggctgcaggtcgac-3' (SEQ ID No. 51) of pZ8 with the DNA molecule having the nucleotide sequence SEQ ID No. 2, while keeping the other nucleotides of pZ8 unchanged.

The nucleotide sequence of the cynX fragment is:

5'-atgctgctggtactggtgctgattggtcttaatatgcgaccactgctcacctccgtcgggccactgctaccgcaattgcgccaggcgagcgg
aatgagctttagcgtggctgccctgttgaccgctctgccggtggttaccatgggcgggctggcgctggccggaagctggcttcatcagcatgtc
agcgaacgtcgcagtgtcgccatcagtctgttgctgattgccgtcggtgcattgatgcgtgagctttacccgcaaagtgcgctgctgcttagcagc
gcactgcttggtggggtggggatcggcatcattcaggcggtgatgccttcggtgattaaacggcggtttcagcagcgcacgccactggtgatgg
ggctgtggtccgcggctctgatgggcggcggtgggcttggtgccgccataacgccctggttagttcaacatagcgaaacctggtatcaaacact
cgcctggtgggcgctgcctgccgttgttgcgctctttgcctggtggtggcaaagcgcccgcgaggtcgcctcttcccacaagacaacaaccact
ccggttcgcgtggtattcactccccgcgcgtggacgctgggtgtttacttcggtctgattaacggcggttacgccagcctgattgcctggttaccc
gctttctatattgagattggtgccagcgcgcagtacagcggttccttactggcattgatgacgcttgggcaagccgcaggagctttgctgatgcct
gctatggctcgccatcaggatcggcgcaaactgttaatgctggcgctggtgttacaactggtggggttctgcggctttatctggctgccgatgcaa
ttgccggtattgtgggcgatggtgtgtgggttaggtctgggcggcgcgtttccgctctgtttgctgctggcgctcgatcactctgtgcaaccggcta
ttgctggcaagctggtggcgtttatgcagggaatcggttttatcatcgccgggcttgccccgtggttttctggcgtgctgcgtagtatcagcggcaa
ttacctgatggactgggcatttcatgcgctgtgcgtcgttgggctgatgatcataaccctgcgttttgcaccagtacgttttccgcagctgtgggtca
aagaggcatga-3' (SEQ ID No. 2).

The recombinant vector pZ8-cynX is transformed into competent DH5α cells. PCR amplification is performed on kanamycin-resistant plates using primers pZ8F/pZ8R (specific sequences are shown in Table 1) to screen for positive transformants. For the specific plasmid transformation method, refer to 1.5 in Step I of the experimental methods described earlier.

After sequencing identification of the PCR products, the positive transformant of the pZ8-cynX plasmid is 1243 bp, and no bands are amplified from the DH5α strain by PCR.

### 2. Construction of engineered strains ARG-pZ8-cynX and W3110-pZ8-cynX

For the preparation of competent cells of the engineered strain ARG10 and the wild-type strain W3110, refer to Step II in the experimental methods described earlier. The recombinant vector pZ8-cynX is transformed into the competent cells of the starting strain ARG10 and the wild-type strain W3110 respectively. PCR amplification is performed on kanamycin-resistant plates using primers pZ8F/pZ8R (specific sequences are shown in Table 1) to screen for positive transformants, thereby constructing the engineered strains ARG-pZ8-cynX and W3110-pZ8-cynX with enhanced cynX gene expression intensity.

The PCR verification of positive strains comprising the pZ8-cynX plasmid should yield a band of 1243 bp, and no bands should be amplified from the starting strains by PCR. The expression level of the cyanate transporter-encoding gene in the recombinant E. coli ARG-pZ8-cynX comprising the pZ8-cynX plasmid is higher than that in the recipient E. coli ARG10. The expression level of the cyanate transporter-encoding gene in the recombinant E. coli W3110-pZ8-cynX comprising the pZ8-cynX plasmid is higher than that in the recipient E. coli W3110.

### C. Construction of the genetically engineered strain ARG:ΔcynX

### 1. Acquisition of the recombinant plasmid pGRB-cynX

### 1) ΔcynX gene editing

Using the genome of Escherichia coli W3110 as a template, upstream homologous arm primers (UP-cynX-S/UP-cynX-A, specific sequences are shown in Table 1) and downstream homologous arm primers (DN-cynX-S/DN-cynX-A, specific sequences are shown in Table 1) are designed according to the upstream and downstream sequences outside the coding region of the cynX gene (NCBI GeneID: 948767). The above fragments are fused by overlapping PCR to obtain ΔcynX (upstream homologous arm of cynX - downstream homologous arm of cynX).

### 2) Construction of pGRB-cynX

A DNA fragment comprising the target sequence (with a nucleotide sequence of 5'-ACtgctaccgcaattgcgcc-3', SEQ ID No. 48) is prepared by annealing primers gRNA-cynX-S and gRNA-cynX-A (specific sequences are shown in Table 1) and then ligated with the plasmid pGRB to construct the recombinant plasmid pGRB-cynX.

The structural description of the recombinant plasmid pGRB-cynX is as follows: it is a recombinant plasmid obtained by replacing the fragment between 5'-gctcagtcctaggtataatactagt-3' (SEQ ID No. 49) and 5'-gttttagagctagaaatagcaagt-3' (SEQ ID No. 31) of pGRB with the DNA molecule having the nucleotide sequence 5'-ACtgctaccgcaattgcgcc-3' (SEQ ID No. 48), while keeping the other nucleotides of pGRB unchanged.

### 2. Construction of the engineered strains ARG: ΔcynX and W3110: Δ cynX

For the preparation of competent cells of the engineered strain ARG10 and the wild-type strain W3110, Step II in the experimental methods described earlier is referred to.

The recombinant vector pGRB-cynX and the ΔcynX fragment are respectively transformed into the competent cells of the starting strain ARG10 and the wild-type strain W3110. PCR amplification is performed on plates resistant to ampicillin (concentration: 100 mg/L) and spectinomycin (concentration: 100 mg/L) using primers UP-cynX-S and DN-cynX-A (specific sequences are shown in Table 1) to screen for positive transformants, thereby constructing the engineered strains ARG: ΔcynX and W3110: ΔcynX with reduced cynX gene expression intensity.

Among them, the length of the upstream homologous arm should be 572 bp, the length of the downstream homologous arm should be 527 bp, and the total length of the overlapping fragment should be 1099 bp. During PCR verification, the length of the PCR-amplified fragment from positive bacteria should be 1099 bp, and the length of the PCR-amplified product from the original bacteria should be 2254 bp. The nucleotide sequence of the ΔcynX fragment is SEQ ID No. 9 in the sequence listing.

The nucleotide sequence of the ΔcynX fragment is:

5'-gtttgtgccataccgctacgccaaccgaccgcagcgtaaccttatttttaaaccatcaggagttccaccatgattcagtcacaaattaaccgca
atattcgtcttgatcttgccgatgccattttgctcagcaaagctaaaaaagatctctcatttgccgagattgccgacggcaccggtctggcagaagc
ctttgtaaccgcggctttgctgggtcagcaggcgcttcctgccgacgccgcccgcctggtcggggcgaagctggatctcgacgaagactccatt
ctactgttgcagatgattccactgcgtggctgcattgatgaccgtattccaactgacccaacgatgtatcgtttctatgaaatgttgcaggtgtacggt
acaaccctgaaagcgttggttcatgagaaatttggcgatggcattattagcgcgattaacttcaaactcgacgttaagaaagtggcggacccgga
aggtggcgaacgtgcggtcatcaccttagatggtaaatatctgccgaccaaaccgttctgacagccatgcgcaaccatcaaaagacgttcacgt
gcgacgcttgttcctgcgctttgttcatgccggatgcggctaatgtagatcgctgaacttgtaggcctgataagcgcagcgtatcaggcaatttttat
aatttaaactgacgattcaactttataatctttgaaataatagtgcttatcccggtcgtttatttcgcgaataacccgacaaggaacgccagccgcca
cgacgtttggtggaatgtcttttgtgacgatactacccgcgccaataacagaattatccccgatggtgacgcctggattaataaccacatgacttcc
gatccagacgttattgccaatcgttatcggaaaagagtacatctcgccgttttttctcaattcatggtgtacagggtgtcccgtaacggaaagagtaa
cgttgggtgcaatcagtacgttatcaccgattgttaccgtgtagtcatcgacaatggttaaattgaaatttgcataaaaattgcggcctatatggatgt
tggaaccgtaagagaaatagacaggcggttctaccc-3' (SEQ ID No. 9)

### D. Fermentative Production of L-arginine Using Engineered Strains with Overexpressed cynX Gene and Engineered Strains with Knocked-out cynX Gene

The strains to be tested are as follows: ARG-cynX, ARG-pZ8-cynX, W3110-cynX, and W3110-pZ8-cynX, which are modified by overexpressing the cynX gene; ARG:ΔcynX, a strain with the cynX gene knocked out; and the starting strain ARG10 (control strain).

The specific fermentation steps are as follows:
1) Slant culture: streak-inoculating the strains stored at -80 °C onto activated slants and culturing them at 37 °C for 12 h, followed by one subculture.
2) Shake-flask seed culture: scraping a loopful of seed from the slant using an inoculating loop and inoculating it into a 500-mL Erlenmeyer flask comprising 30 mL of seed medium. The flask is sealed with nine layers of gauze and it is cultured at 37 °C and 200 rpm for 7 - 10 h.
3) Shake-flask fermentation culture: the culture at an inoculum volume of 10 - 15% of the seed culture volume is inculated into a 500-mL Erlenmeyer flask comprising fermentation medium (final volume of 30 mL). The flask is sealedwith nine layers of gauze and culture it at 37 °C with shaking at 200 r/min. During fermentation, the pH at 7.0 - 7.2 is maintained by adding ammonia water and a 60% (m/v) glucose solution is added to sustain the fermentation process. The fermentation cycle is 26 - 30 h.

The above-mentioned strains are subjected to shake-flask fermentation culture, and the concentration of L-arginine in the fermentation supernatant is determined. The method for determining the L-arginine concentration refers to the industry standard GB 36897-2018.

**Table 7. Shake-flask Fermentation Results of cynX-Engineered Strains**

| Strains | OD₆₀₀ | Arginine (g/L) | Significance Analysis |
|---|---|---|---|
| ARG10 | 38.12±1.53 | 26.3±0.96 | |
| ARG-cynX | 36.78±1.23 | 29.4±0.65 | P<0.01 (**) |
| ARG-pZ8-cynX | 37.57±1.35 | 29.5±0.34 | P<0.01 (**) |
| ARG: Δ cynX | 36.72±1.41 | 24.7±0.13 | P<0.05 (*) |
| W3110 | 47.46±1.35 | 0.001±0.0003 | |
| W3110-cynX | 48.97±1.17 | 0.35±0.001 | P<0.001 (***) |
| W3110-pZ8-cynX | 48.54±1.38 | 0.41±0.001 | P<0.001 (***) |
| W3110: Δ cynX | 48.46±1.35 | 0.001±0.0001 | |

The results are shown in Table 7. After 26 - 30 h of fermentation, enhancing the expression intensity of the cynX gene does not have a significant impact on the growth of the engineered strain ARG10. Meanwhile, in ARG-cynX, the L-arginine concentration increased from 26.3 g/L to 29.4 g/L, and the L-arginine yield increased by 11.8%. In ARG-pZ8-cynX, the L-arginine concentration increased from 26.3 g/L to 29.5 g/L, and the L-arginine yield increased by 12.2%. After reducing the expression intensity of the cynX gene, the L-arginine concentration in ARG: Δ cynX decreased from 26.3 g/L to 24.7 g/L, and the L-arginine yield decreased by 6.1%. W3110 with enhanced cynX expression produced 0.35 - 0.41 g/L of L-arginine.

The results show that enhancing the expression intensity of the cynX gene in Escherichia coli can significantly improve the L-arginine production performance of the engineered strains. Although knocking out the cynX gene does not affect the normal growth of the strains, it significantly reduced the L-arginine production performance of the engineered strains, indicating the promoting effect of the cynX gene on the L-arginine acid-producing ability.

### Example 2: Application of Carbonic Anhydrase (cynT) and Its Encoding Gene in Regulating Arginine Yield

### A. Preparation of Recombinant DNA Fragments

The recombinant fragment for cynT overexpression is composed of the upstream and downstream homologous arms of the cynT gene (ygaY::P_{trc}-cynT). Using the primer design software Primer5, primers for the upstream and downstream homologous arms are designed based on the upstream and downstream sequences of the cynT gene (with an amplification length of approximately 400 - 800 bp). The primers for the upstream homologous arm are UP-ygaY-S/UP-ygaY-A (specific sequences are shown in Table 1); the primers for the downstream homologous arm are cynT-S/cynT-A (specific sequences are shown in Table 1); and another set of primers for the downstream homologous arm are DN-ygaY-S/DN-ygaY-A (specific sequences are shown in Table 1). After separately amplifying the upstream and downstream homologous arms and the target gene fragment by PCR, the recombinant fragment ygaY::P_{trc}-cynT is prepared by overlap PCR. The nucleotide sequence of the recombinant fragment ygaY::P_{trc}-cynT is Sequence 10 in the sequence listing.

The 50 µL PCR system and method are shown in Table 8 below.

**Table 8: PCR Amplification System**

| Component | Volume(µL) |
|---|---|
| DNA template | 1 |
| The upstream primers(10 µmol/L) | 1 |
| The downstream primers(10 µmol/L) | 1 |
| dNTP mixture(10 mmol/L) | 4 |
| 5 × Buffer | 10 |
| HS enzyme (5 U/µL) | 0.5 |
| ddH₂O | 32.5 |

The 50 µL overlap PCR system is shown in Table 9 below.

**Table 9: Overlap PCR Amplification System**

| Component | Volume(µL) |
|---|---|
| Template | 2 |
| The upstream primers for the upstream homologous arm(10 µmol/L) | 1 |
| The downstream primers for the downstream homologous arm(10 µmol/L) | 1 |
| dNTP mixture(10 mmol/L) | 4 |
| 5 × Buffer | 10 |
| HS enzyme (5 U/µL) | 0.5 |
| ddH₂O | 31.5 |

| | |
|---|---|
| Note: The template is composed of equimolar amounts of the amplified fragments of the upstream and downstream homologous arms and the target gene, with a total amount not exceeding 10 ng. | |

PCR reaction conditions (using TaKaRa PrimeSTAR HS enzyme): initial denaturation at 95 °C for 5 min; followed by 30 cycles of denaturation at 98 °C for 10 s, annealing at ((Tm - 3/5) °C) for 15 s, and extension at 72 °C (this enzyme extends approximately 1 kb per minute); a final extension at 72 °C for 10 min; and maintenance at 4 °C.

### 2. Preparation of Electrocompetent Cells comprising pREDCas9

The specific method for preparing electrocompetent cells comprising pREDCas9 refers to Experimental Method II mentioned above.

### 3. Transformation of pGRB and Recombinant DNA Fragments

The pGRB prepared in Experimental Method I mentioned above and the recombinant DNA fragment obtained in Step 1 of Part A of this example are simultaneously electroporated into the electrocompetent cells comprising pREDCas9 from Step 2. After electroporation, the resuscitated culture is spread on LB plates comprising ampicillin (concentration: 100 mg/L) and spectinomycin (concentration: 100 mg/L) and cultured overnight at 32 °C. Colony PCR verification is performed using the specifically designed identification primers UP-ygaY-S and DN-ygaY-A (specific sequences are shown in Table 1), and positive recombinants are screened and the strains are preserved.

### 4. Elimination of Plasmids

### 1) Elimination of pGRB

The positive recombinants obtained in Step 3 are cultured overnight in LB medium comprising 0.2% arabinose. After appropriate dilution, the culture is spread on LB plates with spectinomycin resistance and cultured overnight at 32 °C. The LB plates with ampicillin and spectinomycin resistance are spotted, and single colonies that do not grow on the ampicillin plate but grew on the spectinomycin-resistant plate are selected and the strains are preserved.

### 2) Elimination of the pREDCas9 plasmid

The positive recombinants are transferred to antibiotic-free LB liquid medium and cultured overnight at 42 °C. After appropriate dilution, the culture is spread on antibiotic-free LB plates and cultured overnight at 37 °C. The LB plates with spectinomycin resistance and antibiotic-free LB plates are spotted, and single colonies that do not grow on the spectinomycin-resistant plate but grew on the antibiotic-free plate are selected and the strains are preserved.

### 5. Gene Editing of ygaY::P_{trc}-cynT

1) Using the genome of Escherichia coli W3110 as a template, the upstream homologous arm primers (UP-ygaY-S and UP-ygaY-A) and downstream homologous arm primers (DN-ygaY-S and DN-ygaY-A) are designed according to the upstream and downstream sequences of the ygaY gene (NCBI GeneID: 2847696), and the upstream homologous arm UP-ygaY and downstream homologous arm DN-ygaY are obtained.
2) Using the genome of Escherichia coli W3110 as a template, the primers cynT-S and cynT-A required for amplifying the cynT gene are designed according to the upstream and downstream sequences of the cynT gene (NCBI GeneID: 946548) and the Ptrc promoter sequence, and recombinant fragment 3 is obtained.
3) The above-mentioned upstream homologous arm UP-ygaY, downstream homologous arm DN-ygaY, and recombinant fragment 3 are fused by overlap PCR to obtain ygaY::P_{trc}-cynT (upstream homologous arm-Ptrc-cynT-downstream homologous arm). Among them, the length of the upstream homologous arm should be 616 bp, the length of the Ptrc promoter should be 74 bp, the length of the cynT fragment should be 660 bp, the length of the downstream homologous arm should be 617 bp, the total length of the overlapping fragment should be 1967 bp, and the nucleotide sequence of the overlapping fragment is SEQ ID No.10 in the sequence listing.

The nucleotide sequence of the overlapping fragment is:

5'-cctacaaaccacatcgcacatttcaacaatctatctttcatcccatattcatcaacatccgctattattgatttccagcttaatcatcacctgatgaac
aaaaataatgactaaacctaatcatgagcttagcccggcgctgatcgtgctgatgtctatcgccaccggtctggcggtagccagtaactattacgc

ccagccattgctcgacaccatcgcgcgtaacttttccctttccgccagttcggcaggctttattgttaccgccgcgcagttgggctatgccgcaggt
ctactgtttcttgttcccctcggtgatatgtttgaacgccgccgcctgattgtctcgatgaccttactggcggcatgttgattaccgccagcagtcagt
cgctggcgatgatgatcctcggtacggcattaaccggtttattctcagtcgtggcacaaattctggttccgctggcagcgacgctggcttcaccgg
ataaacgcggcaaagtggttggcactattatgagcggtctgctgttggggatcttgctggcacggacagttgccggattgctggcgaatctcggc
ggctggcgcaccgtcttttgggttgcttcggtgtttgacaattaatcatccggctcgtataatgtgtggaattgtgagcggataacaatttcacacag
gaaacagaccgtgaaagagattattgatggattccttaaattccagcgcgaggcatttccgaagcgggaagccttgtttaaacagctggcgacac
agcaaagcccgcgcacactttttatctcctgctccgacagccgtctggtccctgagctggtgacgcaacgtgagcctggcgatctgttcgttattc
gcaacgcgggcaatatcgtcccttcctacgggccggaacccggtggcgtttctgcttcggtggagtatgccgtcgctgcgcttcgggtatctgac
attgtgatttgtggtcattccaactgtggcgcgatgaccgccattgccagctgtcagtgcatggaccatatgcctgccgtctcccactggctgcgtt
atgccgattcagcccgcgtcgttaatgaggcgcgcccgcattccgatttaccgtcaaaagctgcggcgatggtacgtgaaaacgtcattgctcag
ttggctaatttgcaaactcatccatcggtgcgcctggcgctcgaagaggggcggatcgccctgcacggctgggtctacgacattgaaagcggc
agcatcgcagcttttgacggcgcaacccgccagtttgtgccactggccgctaatcctcgcgtttgtgccataccgctacgccaaccgaccgcag
cgtaattgcttgccgctccaccttttaactacagcgatggtgtcattggtctgtttggacttgcgggagctgccggagcgttgggcgctcgtccggc
gggcggttttgccgataagggcaaatcgcaccacaccacaactttcggtctgctgctgctattactttcatggctggcgatctggtttggtcacactt
ccgtactggcgttgattatcggaatcctggtgctggatctcaccgtgcagggcgtgcatatcactaaccagacggtaatttatcggatacatcctga
tgcgcgcaatcgcctgaccgcaggttacatgactagctactttattggcggtgccgccggttcgctaatttcagcctcagcctggcaacatggcg
gttgggctggcgtttgtctggctggcgcgacgattgccctggttaacttactggtctggtggcgaggttttcatcgtcaggaagccgcaaattaag
caatcagcaaccgttttcgtggctttacacttataagggtgttaagaagcccatcagtctgataaggttaagatattcattcagtctatttataatattaa
caatcgttaagcgtacactctatggaaagccctactcc-3' (SEQ ID No.10).

4) Construction of pGRB-ygaY: A DNA fragment comprising the target sequence obtained by annealing the primers gRNA-ygaY-S and gRNA-ygaY-A is ligated with the plasmid pGRB to construct the recombinant plasmid pGRB-ygaY.

The structural description of the recombinant plasmid pGRB-ygaY is as follows: it is a recombinant plasmid obtained by replacing the fragment between 5'-gctcagtcctaggtataatactagt-3' (SEQ ID No.49) and 5'-gttttagagctagaaatagcaagt-3' (SEQ ID No.31) of pGRB with a DNA molecule with the nucleotide sequence 5'-ctcaactacccacagttgtt-3' (SEQ ID No.46), while keeping the other nucleotides of pGRB unchanged.

5) The competent cells of the engineered strain ARG10 (derived from CN 110964683 B) and the wild-type strain W3110 are prepared, referring to Experimental Method II mentioned above.

The PCR verification of the positive strain after the integration of ygaY::P_{trc}-cynT should be 1967 bp, and the PCR amplification of the original strain should be 1424 bp. The nucleotide sequence of the ygaY::P_{trc}-cynT fragment is SEQ ID No.10 in the sequence listing.

Successively the engineered strains ARG-cynT and W3110-cynT with enhanced cynT gene expression intensity are constructed.

Step 1: constructing the pGRB-cynT vector; Step 2: amplifying the ygaY::P_{trc}-cynT integration fragment by PCR; Step 3: preparing the competent cells of ARG10 and W3110 and transform them with pREDCas9; Step 4: preparing the competent cells of the target strains ARG10 and W3110 comprising pREDCas9 and transform them with the pGRB-cynT vector and the ygaY::P_{trc}-cynT integration fragment; Step 5: Since the strains ARG-cynT and W3110-cynT integrated with ygaY::P_{trc}-cynT contain the pGRB-cynT vector, eliminating the pGRB-cynT vector; Step 6: Eliminating the pREDCas9 vector from the target strains ARG-cynT and W3110-cynT after the elimination of the pGRB-cynT vector to obtain the engineered strains ARG-cynT and W3110-cynT that do not contain the pGRB-cynT and pREDCas9 vectors and have enhanced cynT gene expression intensity. The specific steps refer to Steps 1 to 5 of this example.

The expression level of the carbonic anhydrase-encoding gene cynT in the engineered strain ARG-cynT is higher than that in the recipient Escherichia coli ARG10. The expression level of the carbonic anhydrase-encoding gene cynT in the engineered strain W3110-cynT is higher than that in the recipient Escherichia coli W3110.

### B. Construction of genetically engineered strains ARG-pZ8-cynT and W3110-pZ8-cynT

A low-copy recombinant expression vector pZ8-cynT is constructed using the shuttle expression vector pZ8. This vector is then introduced into the engineered strain ARG10 and the wild-type strain W3110. The tac promoter on the pZ8 vector is used to initiate the expression of cynT, aiming to enhance the yield of L-arginine.

### 1. Construction of recombinant expression vector pZ8-cynT

Primers cynT-pS and cynT-pA (specific sequences are shown in Table 1) required for amplifying the cynT gene are designed based on the upstream and downstream sequences of the cynT gene (NCBI GeneID: 946548). Using the Escherichia coli W3110 genome as a template, the cynT gene is amplified by PCR with the primers cynT-pS and cynT-pA, and then purified and recovered. The nucleotide sequence of the obtained cynT fragment is SEQ ID No.3 in the sequence listing. The PCR system and program are detailed in Step I of the experimental methods described earlier.

After the pZ8 vector is digested with EcoR I and Sal I and purified, the above-mentioned purified cynT PCR product is recombined with the linearized pZ8 vector to obtain the recombinant vector pZ8-cynT of cynT and pZ8.

The structural description of the recombinant vector pZ8-cynT is as follows: it is a recombinant plasmid obtained by replacing the fragment between 5'-tgagcggataacaatttcacacaggaaacagaattc-3' (SEQ ID No.50) and 5'-tctcatccgccaaaacagaagcttggctgcaggtcgac-3' (SEQ ID No.51) of pZ8 with the DNA molecule having the nucleotide sequence SEQ ID No.3, while keeping the other nucleotides of pZ8 unchanged.

The nucleotide sequence of the cynT fragment is as follows:

5'-gtgaaagagattattgatggattccttaaattccagcgcgaggcatttccgaagcgggaagccttgtttaaacagctggcgacacagcaaag
cccgcgcacactttttatctcctgctccgacagccgtctggtccctgagctggtgacgcaacgtgagcctggcgatctgttcgttattcgcaacgc
gggcaatatcgtcccttcctacgggccggaacccggtggcgtttctgcttcggtggagtatgccgtcgctgcgcttcgggtatctgacattgtgatt
tgtggtcattccaactgtggcgcgatgaccgccattgccagctgtcagtgcatggaccatatgcctgccgtctcccactggctgcgttatgccgat
tcagcccgcgtcgttaatgaggcgcgcccgcattccgatttaccgtcaaaagctgcggcgatggtacgtgaaaacgtcattgctcagttggctaa
tttgcaaactcatccatcggtgcgcctggcgctcgaagaggggcggatcgccctgcacggctgggtctacgacattgaaagcggcagcatcgc
agcttttgacggcgcaacccgccagtttgtgccactggccgctaatcctcgcgtttgtgccataccgctacgccaaccgaccgcagcgtaa-3'
(SEQ ID No.3).

The recombinant vector pZ8-cynT is transformed into DH5α competent cells. PCR amplification is carried out on kanamycin-resistant plates using primers pZ8F/pZ8R (specific sequences are shown in Table 1) to screen for positive transformants. The specific plasmid transformation method is detailed in Step I, 1.5 of the experimental methods described earlier.

After sequencing identification of the PCR products, the PCR product length of the positive transformant of the pZ8-cynT plasmid should be 748 bp, and no band is amplified from the PCR of the DH5α strain.

### 2. Construction of engineered strains ARG-pZ8-cynT and W3110-pZ8-cynT

The preparation of competent cells of the engineered strain ARG10 and the wild-type strain W3110 is detailed in Step II of the experimental methods described earlier. The recombinant vector pZ8-cynT is transformed into the competent cells of the starting strains ARG10 and the wild-type strain W3110 respectively. PCR amplification is carried out on kanamycin-resistant plates using primers pZ8F/pZ8R (specific sequences are shown in Table 1) to screen for positive transformants, thereby constructing the engineered strains ARG-pZ8-cynT and W3110-pZ8-cynT with enhanced expression intensity of the cynT gene.

The PCR product length of the positive strains comprising the pZ8-cynT plasmid should be 748 bp, and no band is amplified from the PCR of the starting strains. The expression level of the carbonic anhydrase-encoding gene in the recombinant E. coli ARG-pZ8-cynT comprising the pZ8-cynT plasmid is higher than that in the recipient E. coli ARG10. The expression level of the carbonic anhydrase-encoding gene in the recombinant E. coli W3110-pZ8-cynT comprising the pZ8-cynT plasmid is higher than that in the recipient E. coli W3110.

### C. Construction of genetically engineered strain ARG:ΔcynT

### 1. Acquisition of recombinant plasmid pGRB-cynT

### 1) Δ cynT gene editing

Using the Escherichia coli W3110 genome as a template, upstream homologous arm primers (UP-cynT-S and UP-cynT-A, specific sequences are shown in Table 1) and downstream homologous arm primers (DN-cynT-S and DN-cynT-A) are designed according to the upstream and downstream sequences outside the coding region of the cynT gene (NCBI GeneID: 946548). The above fragments are fused by overlapping PCR to obtain ΔcynT (upstream homologous arm of cynT - downstream homologous arm of cynT).

### 2) Construction of pGRB-cynT

A DNA fragment comprising the target sequence (the target sequence is the nucleotide sequence 5'-atttgtggtcattccaactg-3' (SEQ ID No.47)) is prepared by annealing primers gRNA-cynT-S and gRNA-cynT-A and then ligated with the plasmid pGRB to construct the recombinant plasmid pGRB-cynT.

The structural description of the recombinant plasmid pGRB-cynT is as follows: it is a recombinant plasmid obtained by replacing the fragment between 5'-gctcagtcctaggtataatactagt-3' (SEQ ID No.49) and 5'-gttttagagctagaaatagcaagt-3' (SEQ ID No.31) of pGRB with the DNA molecule having the nucleotide sequence 5'-Atttgtggtcattccaactg-3' (SEQ ID No.47), while keeping the other nucleotides of pGRB unchanged.

### 2. Construction of engineered strains ARG: Δ cynT and W3110:ΔcynT

The preparation of competent cells of the engineered strain ARG10 and the wild-type strain W3110 is detailed in Step II of the experimental methods described earlier.

The recombinant vector pGRB-cynT and the ΔcynT fragment are respectively transformed into the competent cells of the starting strains ARG10 and the wild-type strain W3110. PCR amplification is carried out on kanamycin-resistant plates using primers UP-cynT-S/DN-cynT-A (specific sequences are shown in Table 1) to screen for positive transformants, thereby constructing the engineered strains ARG:ΔcynT and W3110:ΔcynT with reduced expression intensity of the cynT gene.

Among them, the length of the upstream homologous arm should be 543 bp, the length of the downstream homologous arm should be 519 bp, and the total length of the overlapping fragment should be 1062 bp. During PCR verification, the length of the PCR-amplified fragment of the positive strain with successful cynT knockout should be 1062 bp, and the length of the PCR-amplified product of the original strain should be 1722 bp. The nucleotide sequence of the ΔcynT fragment is SEQ ID No.11 in the sequence listing.

The nucleotide sequence of the ΔcynT fragment is:

5'-aatcccaacgtccaactcgtcgcggcaaagcatatcctcgattttctcctgcgacatttcctgtagctggagcgtgatgctgggatagcgcgc
atagaaatccgccattaaggggccgataaagtagctcgtaaaggtgggggtgacggcgatacgcagcgatcctcgcgtcagatcggcaacat
catgaatcgcccgtttacccgcccccagttcctgtaacgcccggctggcgtactgtcgccagacttctcctgcatcagtgagacgaatcgttcgc
ccgctacggtcaaacagcggcacgcctaaactctcctctaactggcgaatctgctgggaaagcgcaggttgggagacgtgcaacgcactggc
ggcacgggtgaagctgccatgttcagccacggcaagaaaataattgatatgtcgagagagcattcgcaacctataagtaaatccaatggaactc
atcataaatgagacttttaccttatgacaatcggcgagtagtctgcctctcattccagagacagacagaggttaacgccttatttttaaaccatcagg
agttccaccatgattcagtcacaaattaaccgcaatattcgtcttgatcttgccgatgccattttgctcagcaaagctaaaaaagatctctcatttgcc
gagattgccgacggcaccggtctggcagaagcctttgtaaccgcggctttgctgggtcagcaggcgcttcctgccgacgccgcccgcctggtc
ggggcgaagctggatctcgacgaagactccattctactgttgcagatgattccactgcgtggctgcattgatgaccgtattccaactgacccaac
gatgtatcgtttctatgaaatgttgcaggtgtacggtacaaccctgaaagcgttggttcatgagaaatttggcgatggcattattagcgcgattaactt
caaactcgacgttaagaaagtggcggacccggaaggtggcgaacgtgcggtcatcaccttagatggtaaatatctgccgaccaaaccgttctg
acagccatgcgcaaccatc-3' (SEQ ID No.11).

### D. Fermentative production of L-arginine using engineered strains with overexpressed cynT gene and engineered strains with knocked-out cynT gene

The strains to be tested are as follows: ARG-cynT, ARG-pZ8-cynT, W3110-cynT, and W3110-pZ8-cynT modified by overexpressing the cynT gene, the cynT gene knockout strain W3110: Δ cynT, and the starting strains ARG10 and W3110 (control strains).

The specific fermentation steps are as follows:
1) Slant culture: streak-inoculating the strains stored at -80 °C onto activated slants and culturing at 37 °C for 12 h, and then subculturing once.
2) Shake-flask seed culture: scraping a loopful of seed from the slant with an inoculating loop and inoculating it into a 500-mL Erlenmeyer flask comprising 30 mL of seed medium. The flask is sealed with nine layers of gauze and culture at 37 °C and 200 rpm for 7 - 10 h.
3) Shake-flask fermentation culture: Inoculating at an inoculum size of 10 - 15% of the volume of the seed culture into a 500-mL Erlenmeyer flask comprising fermentation medium (final volume of 30 mL). The flask is sealed with nine layers of gauze and it is cultured at 37 °C with shaking at 200 r/min. During fermentation, the pH at 7.0 - 7.2 is maintained by adding ammonia water and the fermentation process is maintained by adding a 60% (m/v) glucose solution. The fermentation cycle is 26 - 30 h.

The above-mentioned strains are subjected to shake-flask fermentation culture, and the concentration of L-arginine in the fermentation supernatant is determined. The method for determining the concentration of L-arginine refers to the industry standard GB 36897-2018.

**Table 10. Shake-flask fermentation results of cynT engineered strains**

| Strains | OD₆₀₀ | Arginine (g/L) | Significance Analysis |
|---|---|---|---|
| ARG10 | 38.48+1.28 | 26.3+0.96 | |
| ARG-cynT | 37.05±1.55 | 30.2±0.35 | P<0.01 (**) |
| ARG-pZ8-cynT | 38.87±1.43 | 30.9±0.21 | P<0.01 (**) |
| ARG: Δ cynT | 38.54±1.36 | 24.4±0.02 | P<0.05 (*) |
| W3110 | 47.46±1.35 | 0.001±0.0003 | |
| W3110-cynT | 49.73±1.17 | 0.4±0.02 | P<0.001 (***) |
| W3110-pZ8-cynT | 48.94±1.38 | 0.4±0.02 | P<0.001 (***) |
| W3110: Δ cynT | 47.76±1.43 | 0.001±0.0001 | |

The results are shown in Table 10. After 26 - 30 h of fermentation, enhancing the expression intensity of the cynT gene does not have a significant impact on the growth of the engineered strain ARG10. The concentration of L-arginine in ARG-cynT increased from 26.3 g/L to 30.2 g/L, and the yield of L-arginine increased by 14.8%; while the concentration of L-arginine in ARG-pZ8-cynT increased from 26.3 g/L to 30.9 g/L, and the yield of L-arginine increased by 17.5%; the concentration of L-arginine in ARG: Δ cynT decreased from 26.3 g/L to 24.4 g/L, and the yield of L-arginine decreased by 7.2%; and W3110 with enhanced cynT expression also produced 0.4 g/L of L-arginine.

The results show that enhancing the expression intensity of the cynT gene in Escherichia coli can significantly improve the L-arginine production performance of the engineered strains. Knocking out the cynT gene does not affect the normal growth of the strains but significantly reduced the L-arginine production performance of the engineered strains, which once again demonstrates the role of the cynT gene in enhancing the L-arginine production capacity.

### Industrial Application

In this disclosure, the native promoters of the Escherichia coli cynX gene or cynT gene are replaced with Ptrc and integrated into the pseudogene site of ygaY on the genome. The cynX gene or cynT gene is then driven by the strong promoter Ptrc to enhance the expression intensity of the cynX gene or cynT gene, resulting in a more cost-effective fermentative production of L-arginine. Using an L-arginine engineered strain ARG10 as the starting strain (Patent: ZL201911211097.X), enhancing the expression intensity of the cynX gene increased the accumulated concentration of L-arginine in this strain from 26.3 g/L to 29.4 g/L, with an 11.8% increase in the yield of L-arginine; enhancing the expression intensity of the cynT gene increased the accumulated concentration of L-arginine in this strain from 26.3 g/L to 30.2 g/L, with a 14.8% increase in the yield of L-arginine.

## Claims

1. A recombinant enterobacteria, wherein the recombinant enterobacteria is a recombinant strain obtained by overexpressing a cyanate transporter-encoding gene and/or overexpressing a carbonic anhydrase-encoding gene in recipient enterobacteria.

2. The recombinant enterobacteria according to claim 1, wherein the cyanate transporter gene is derived from escherichia coli, and the carbonic anhydrase gene is derived from escherichia coli.

3. The recombinant enterobacteria according to claim 1 or 2, wherein:
the cyanate transporter is any one of the following proteins:
A1) a protein with an amino acid sequence encoded by the cyanate transporter-encoding gene is the protein set forth in SEQ ID No.4;
A2) a protein obtained by substitution and/or deletion and/or addition of amino acid residues in the amino acid sequence of A1), having more than 80% identity with the protein of A1) and having cyanate transporter activity;
A3) a fusion protein having cyanate transporter activity obtained by connecting a tag to n-terminus and/or c-terminus of A1) or A2);
the carbonic anhydrase is any one of the following proteins:
B1) a protein with amino acid sequence encoded by the carbonic anhydrase-encoding gene is the protein set forth in SEQ ID No.5;
B2) a protein obtained by substitution and/or deletion and/or addition of amino acid residues in the amino acid sequence of B1), having more than 80% identity with the protein of B1) and having carbonic anhydrase activity;
B3) a fusion protein having carbonic anhydrase activity obtained by connecting a tag to n-terminus and/or c-terminus of B1) or B2).

4. The recombinant enterobacteria according to any one of claims 1-3, wherein an encoding gene of the cyanate transporter is any one of the following:
C1) a DNA molecule with the nucleotide sequence of SEQ ID No.2;
C2) a DNA molecule having more than 80% identity with the nucleotide sequence defined in C1) and encodes the cyanate transporter;
C3) a DNA molecule hybridizing with the nucleotide sequence defined in any one of D1)-C2) under stringent conditions and encodes the cyanate transporter;
an encoding gene of the carbonic anhydrase is any one of the following:
D1) a DNA molecule with the nucleotide sequence of SEQ ID No.3;
D2) a DNA molecule having more than 80% identity with the nucleotide sequence defined in d1) and encodes the carbonic anhydrase;
D3) a DNA molecule hybridizing with the nucleotide sequence defined in any one of d1)-d2) under stringent conditions and encodes the carbonic anhydrase.

5. A method for constructing the recombinant enterobacteria according to any one of claims 1-4, the method comprising:
Q1) regulating the arginine yield of microorganisms by regulating the expression of the encoding gene of the cyanate transporter according to any one of claims 1-4, or regulating the activity or content of the cyanate transporter, to obtain microorganisms with a changed arginine yield;
Q2) regulating the arginine yield of microorganisms by regulating the expression of the encoding gene of the carbonic anhydrase according to any one of claims 1-4, or regulating the activity or content of the carbonic anhydrase, to obtain microorganisms with a changed arginine yield.

6. The method according to claim 5, wherein the method for regulating the expression of the encoding gene of the cyanate transporter or regulating the activity or content of the cyanate transporter is any one of the following:
M1) introducing the encoding gene of the cyanate transporter into target microorganisms;
M2) introducing the encoding gene with the amino acid sequence of SEQ ID No.4 into target microorganisms;
the method for regulating the expression of the encoding gene of the carbonic anhydrase or regulating the activity or content of the carbonic anhydrase is any one of the following:
N1) introducing the encoding gene of the carbonic anhydrase into target microorganisms;
N2) introducing the encoding gene with the amino acid sequence of SEQ ID No.5 into target microorganisms.

7. Use of the enterobacteria according to any one of claims 1-4 in any one of the following:
E1) use in regulating the arginine yield of microorganisms;
E2) use in preparing arginine;
E3) use in constructing genetically engineered microorganisms for arginine.

8. Use of the enterobacteria according to any one of claims 1-4 in producing arginine or foods, pharmaceuticals, and/or feeds comprising arginine.

9. Use of the method according to claim 5 or 6 in any one of the following:
E1) use in regulating the arginine yield of microorganisms;
E2) use in preparing arginine;
E3) use in constructing genetically engineered microorganisms for arginine.

10. Use of the method according to claim 5 or 6 in producing arginine or foods, pharmaceuticals, and/or feeds comprising arginine.

11. Use of proteins, wherein the proteins are protein A or protein B,
the protein A is a cyanate transporter with the amino acid sequence of SEQ ID No.4, and the protein B is a carbonic anhydrase with the amino acid sequence of SEQ ID No.5;
the use is any one of the following:
F1) use in regulating the arginine yield of microorganisms;
F2) use in preparing arginine;
F3) use in constructing genetically engineered microorganisms for arginine.

12. A nucleic acid molecule, wherein the nucleic acid molecule is nucleic acid molecule A or nucleic acid molecule B; the nucleic acid molecule A encodes the cyanate transporter in the enterobacteria according to any one of claims 1-4; the nucleic acid molecule B encodes the carbonic anhydrase in the enterobacteria according to any one of claims 1-4.

13. An expression cassette, wherein the expression cassette is expression cassette A or expression cassette B, the expression cassette A comprises the nucleic acid molecule B according to claim 12, and the expression cassette B comprises the nucleic acid molecule b according to claim 12.

14. A recombinant vector, wherein the expression cassette is recombinant vector A or recombinant vector B, the recombinant vector a comprises the nucleic acid molecule A according to claim 12 or comprises the expression cassette according to claim 13, and the recombinant vector b comprises the nucleic acid molecule B according to claim 12 or comprises the expression cassette according to claim 13.

15. A whole-cell catalyst, wherein the whole-cell catalyst is whole-cell catalyst A or whole-cell catalyst B, the whole-cell catalyst A comprises the nucleic acid molecule A according to claim 12, and the whole-cell catalyst B comprises the nucleic acid molecule B according to claim 12.
